# EUROPÄISCHE PATENTSCHRIFT

(11) **EP 3 229 916 B1**
(45) Veröffentlichungstag und Bekanntmachung des Hinweises auf die Patenterteilung: **22.07.2020**
(21) Anmeldenummer: 15790098.6
(22) Anmeldetag: 02.11.2015
(51) Int. Cl.: A61Q 5/06, A61K 8/81, A61K 8/87

(54) **MITTEL UND VERFAHREN ZUR PFLEGE KERATINHALTIGER FASERN**
COMPOSITIONS AND METHODS FOR KERATIN-CONTAINING FIBER CARE
COMPOSITIONS ET METHODES POUR LE SOIN DES FIBRES KERATINIQUES

(30) Priorität: 10.12.2014 DE 102014225436
(43) Veröffentlichungstag der Anmeldung: 18.10.2017
(73) Patentinhaber: Henkel AG & Co. KGaA, 40589 Düsseldorf (DE)
(72) Erfinder: FÖRSTER, Thomas, 40591 Düsseldorf (DE); BAYERSDÖRFER, Rolf, 22589 Hamburg (DE); KNAPPE, Thorsten, 22869 Schenefeld (DE)
(86) Internationale Anmeldenummer: PCT/EP2015/075392
(87) Internationale Veröffentlichungsnummer: WO 2016/091468

(56) Entgegenhaltungen:
- WO-A1-99/66888
- WO-A2-2011/076490
- US-A- 3 372 840
- US-A1- 2002 074 349
- US-A1- 2009 098 079
- US-A1- 2010 297 036
- US-A1- 2013 018 333

## Beschreibung

Die Anmeldung betrifft das technische Fachgebiet der Pflege keratinhaltiger Fasern, insbesondere menschlicher Haare. Gegenstand der Anmeldung sind spezifische haarkosmetische Formulierungen, welche sich zur Applikation auf keratinhaltige Fasern mittels eines Entspannungsverdampfungsverfahrens eignen. Darüber hinaus sind die Verwendung dieser haarkosmetischen Formulierungen in Vorrichtungen zur Entspannungsverdampfung und Verfahren zur temporären Umformung keratinhaltiger Fasern Gegenstand der vorliegenden Anmeldung.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, welche sich bei vielen Haartypen nur unter Verwendung von Haarverformungsverfahren aufrechterhalten lassen.

Für die permanente oder temporäre Formgebung menschlicher Haare werden eine Reihe unterschiedlicher Techniken, beispielsweise Gestaltungen wie Lockung, Glättung, Toupierung oder auch Festigung, eingesetzt. Diese Techniken beruhen auf dem Einsatz festigender kosmetischer Mittel, so genannter Stylingmittel, wie Haarsprays, Haarwachse, Haargele, Haarfestiger, Fönwellen, Stylingsprays.

Alternativ oder in Ergänzung zu der Behandlung mit festigenden kosmetischen Mitteln können die Haare einer thermischen Behandlung unterzogen werden.

Um glattes Haar zu wellen oder zu kräuseln, werden die Haare unter Wärmeeinwirkung, beispielsweise mit Hilfe eines Föhns mit Diffusor oder eines Lockenstabs, verformt. Dabei wird das glatte Haar um den aufgeheizten Lockenstab oder den Diffusor gewickelt, wobei wiederum Temperaturen bis hin zu 250°C erreicht werden. In diesem Verfahren kommen zur Verbesserung des Stylingergebnisses in der Regel Stylingsprays zum Einsatz, die vor der eigentlichen Temperaturbehandlung auf das Haar aufgebracht werden.

Zur Glättung der Haare werden spezielle Glätteisen eingesetzt. Glätteisen weisen zwei parallel angeordnete Metall- oder Keramikplatten auf, durch welche die Haare, nach Aufheizen der Platten, gezogen werden. Handelsübliche Glätteisen lassen sich auf Temperaturen im Bereich von 150-250 °C aufheizen. Ziel der Glätteisenanwendung ist es, gewelltes bis gelocktes Haar thermisch/physikalisch zu glätten. Sollen Haare durch ein Glätteisen geglättet werden, wird auf das Haar üblicherweise vorher als Stylingmittel für Glätteisen, ein Stylingspray, auch Glätteisenspray bezeichnet, aufgetragen. Das Spray unterstützt dabei das Gleiten des Eisens sowie die Glättung des Haares.

Zum Schutz des Haares vor den Auswirkungen der für die Haarverformung eingesetzten hohen Temperaturen können Hitzeschutzmittel eingesetzt werden. Bei der Anwendung dieser Hitzeschutzmittel hat insbesondere die Sprühapplikation entsprechender kosmetischer Zubereitungen eine große Bedeutung, wobei die Zubereitungen in der Regel als Pumpsprays oder Aerosolsprays appliziert werden (so wie zum Beispiel in der WO 2011/076490). Hierzu werden die kosmetischen Zubereitungen in einer Abgabevorrichtung konfektioniert, aus denen sie entweder mittels mechanischer Krafteinwirkung oder mit Hilfe eines Treibmittels über ein Ventil versprüht werden. Beide Methoden haben offensichtliche Nachteile. Während Pumpsprays sich in der Regel nicht für eine lang anhaltende gleichmäßige Sprühapplikation haarkosmetischer Zubereitungen eignen, basieren Aersolsprays auf dem Einsatz von Treibmitteln oder Treibgasen, die einerseits keine kosmetische Wirkung entfalten und von denen andererseits bei unsachgemäßer Handhabung eine Gefährdung der Verbraucher ausgehen kann.

Vor diesem Hintergrund besteht ein Bedarf nach alternativen Wegen zur Zerstäubung haarkosmetischer Zubereitungen. Als ein solches alternatives Sprühverfahren hat sich die Entspannungsverdampfung erwiesen. Bei diesem Verfahren, das beispielsweise in der internationalen Patentanmeldung WO 2001/83071 A1 (Henkel) beschrieben wird, wird eine flüssige oder pastöse lösungsmittelhaltige Zusammensetzung in einem abgeschlossenen Raum auf eine Temperatur erhitzt, die über dem Siedepunkt des Lösungsmittels liegt, wodurch in der Zusammensetzung ein Überdruck erzeugt wird. Bei Entspannung (Drosselung) des Drucks verdampft die Flüssigkeit und kann nachfolgend beispielsweise mittels einer geeigneten Düse zerstäubt werden.

Auch wenn die Entspannungsverdampfung also grundsätzlich für die Sprühapplikation haarkosmetischer Zubereitungen geeignet ist, so kann gleichzeitig doch nicht jede haarkosmetische Zubereitung mittels eines Entspannungsverdampfungsverfahrens zerstäubt werden. Dies liegt einerseits an der für die Entspannungsverdampfung notwendigen Erhitzung der kosmetischen Zubereitung, andererseits an den Spezifika der durch Entspannungsverdampfung erzeugten Sprühnebel, beispielsweise der erzeugten Tröpfchengröße und Tröpfchendichte im Sprühnebel.

Aufgabe der vorliegenden Erfindung war es daher, spezifische haarkosmetische Zubereitungen zur temporären Verformung keratinhaltiger Fasern zur Verfügung zu stellen, welche sich aufgrund ihrer chemischen und physikalischen Eigenschaften zur zielgerichteten Sprühapplikation mittels einer Vorrichtung zur Entspannungsverdampfung eignen. Weiterhin sollen die Zubereitungen geeignet sein, nach einer Applikation mittels eines Entspannungsverfahrens einen hohe Pflegewirkung zu realisieren. Es hat sich gezeigt, dass zur Lösung dieser Aufgabe aus der Vielzahl bekannten haarkosmetisch wirksamen Polymerzubereitungen insbesondere Zubereitungen mit hohem Lösungsmittelanteil auf Grundlage filmbildender Polymere geeignet sind.

Ein erster Gegenstand der vorliegenden Erfindung ist somit ein kosmetisches Produkt, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer;
b) eine Vorrichtung zur Entspannungsverdampfung der kosmetischen Zubereitung a), wobei der Gewichtsanteil der Komponenten a) und b) am Gesamtgewicht der kosmetischen Zubereitung a) mehr als 92% beträgt.

Dabei ist die Vorrichtung zur Entspannungsverdampfung im Sinne der vorliegenden Erfindung wie in den Ansprüchen beschrieben. Die kosmetische Zubereitung a) ist flüssig. Die kosmetische Zubereitung a) kann als Lösung oder Dispersion, beispielsweise als Emulsion oder Suspension vorliegen. Bevorzugte kosmetische Zubereitungen a) liegen in Form einer Lösung oder einer Suspension vor.

Die erfindungsgemäße kosmetische Zubereitung enthält als ersten wesentlichen Bestandteil mindestens 88 Gew.-% eines polaren Lösungsmittels a1). Bevorzugte kosmetische Produkte sind dadurch gekennzeichnet, dass der Gewichtsanteil des polaren Lösungsmittels a1) am Gesamtgewicht der kosmetischen Zubereitung a) mindestens 80 Gew.-%, bevorzugt mindestens 85 Gew.-%, besonders bevorzugt mindestens 90 Gew.-% und insbesondere mindestens 95 Gew.-% beträgt. Entsprechende Mittel zeichnen sich durch eine gute kosmetische Wirkung bei gleichzeitig guter Applizierbarkeit aus.

Zur Verbesserung der Anwendungseigenschaften erfindungsgemäßer kosmetischer Zubereitungen bei gleichzeitiger Minimierung der thermischen Belastung etwaiger Wirk- oder Hilfsstoffe im Verlauf des Entspannungsverdampfungsverahrens, hat es sich als vorteilhaft erwiesen, polare Lösungsmittel a1) einzusetzen, welche eine Siedetemperatur (20°C, 1013 mbar) zwischen 50 und 110°C, vorzugsweise zwischen 70 und 105°C aufweisen. Als besonders geeignet haben sich dabei Ethanol und Wasser erwiesen, welche aus diesem Grund als polare Lösungsmittel a1) bevorzugt werden.

Besonders bevorzugte polare Lösungsmittel a1) oder Lösungsmittelsysteme sind
- polare Lösungsmittel a1), umfassend mehr als 80 Gew.-%, vorzugsweise mehr als 85 Gew.-% und insbesondere mehr als 90 Gew.-% Wasser, jeweils bezogen auf das Gesamtgewicht des polaren Lösungsmittels;
- polare Lösungsmittel a1), umfassend Wasser und Ethanol, wobei das Gewichtsverhältnis von Wasser zu Ethanol 50:1 bis 1:1, bevorzugt 40:1 bis 5:1 und insbesondere 25:1 bis 10:1 beträgt.

Ein zweiter wesentlicher Bestandteil erfindungsgemäßer kosmetischer Zusammensetzungen ist das filmbildende Polymer a2). Als filmbildende Polymere a2) eignen sich permanent als auch temporär kationische, anionische, nichtionische oder amphotere Polymere. Diese filmbildenden Polymere können synthetischen oder natürlichen Ursprungs sein. In Bezug auf die Herstellbarkeit, Applizierbarkeit und kosmetische Wirkung erfindungsgemäßer kosmetischer Zusammensetzungen hat es sich als vorteilhaft erwiesen, wenn der Gewichtsanteil des filmbildenden Polymers am Gesamtgewicht der kosmetischen Zubereitung 0,1 bis 10 Gew.-%, vorzugsweise 0,2 bis 8,0 Gew.-% und insbesondere 0,4 bis 5,0 Gew.-% beträgt.

Beispiele für gebräuchliche filmbildende Polymere a4) sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Aufgrund ihrer kosmetischen Wirkung bilden die nichtionischen Polymere, vorzugsweise Polymere aus der Gruppe der Polyvinylpyrrolidone und Vinylpyrrolidon/Vinylacetat-Copolymere, insbesondere der Vinylpyrrolidon/Vinylacetat-Copolymere, eine erste Gruppe besonders bevorzugter filmbildender Polymere a2).

Bevorzugte Polyvinylpyrrolidone (INCI-Bezeichnung: PVP) weden beispielsweise unter der Bezeichnung Luviskol® (BASF) vertrieben.

Die Vinylpyrrolidon/Vinylacetat-Copolymere sind auf die Monomere i) Vinylpyrrolidon und ii) Vinylacetat sowie gegebenenfalls weitere Monomere zurückführbar. Bevorzugte Vinylpyrrolidon/Vinylacetat-Copolymere bestehen zu mindestens 90 Gew.-%, vorzugsweise zu mindestens 95 Gew.-% und insbesondere zu mindestens 97 Gew.-% aus den Monomeren i) Vinylpyrrolidon und ii) Vinylacetat. Besonders bevorzugte Copolymere e) wurden ausschließlich aus den Monomeren i) Vinylpyrrolidon und ii) Vinylacetat erhalten.

In den Vinylpyrrolidon-Vinylacetat-Copolymeren können Vinylpyrrolidon und Vinylacetat in unterschiedlichen Verhältnissen vorliegen. Vinylpyrrolidon-Vinylacetat-Copolymere mit einem höheren Vinylpyrrolidonanteil sind wasserlöslich, etwa ab einem Vinylpyrrolidon-Anteil von 60%. Vinylpyrrolidon-Vinylacetat-Copolymere mit niedrigerem Vinylpyrrolidon-Anteil können aber in Ethanol gelöst werden. Es ist erfindungsgemäß bevorzugt, wenn das Vinylpyrrolidon-Vinylacetat-Copolymer a2) ein Gewichtsverhältnis von Vinylpyrrolidon zu Vinylacetat von 30:70 bis 70:30, aufweist.

Die zuvor beschriebenen Vinylpyrrolidon/Vinylacetat-Copolymere werden beispielsweise unter der Bezeichnung Luviskol® VA 37, Luviskol® VA 55, Luviskol® VA 64, Luviskol® VA 73 (INCI Bezeichnung: VP/VA Copolymer; CAS Nummer 25086-89-9), jeweils von der Firma BASF SE vertrieben.

Eine zweite Gruppe filmbildender Polymere a2) mit besonders vorteilhaften kosmetischen Eigenschaften bilden die Polyurethane. Unter dem Begriff "Polyurethanpolymere" werden Polymere verstanden, bei welchen mindestens zwei Monomere, bevorzugt mindestens drei Monomere, über eine Urethan-Gruppierung -NH-CO-O- verknüpft sind. Erfindungsgemäß sind auch solche Polyurethanpolymere eingeschlossen, welche herstellungsbedingt harnstoffgruppenhaltige Wiederholungseinheiten -NH-C(O)-NH- aufweisen, wie sie insbesondere bei der Umsetzung von isocyanatterminierten Prepolymeren mit aminogruppenhaltigen Verbindungen gebildet werden. Erfindungsgemäß besonders bevorzugt sind Polyurethanpolymere, bei welchen alle Monomere über Urethan-Gruppierungen und/oder harnstoffgruppenhaltige Gruppierungen verknüpft sind. Derartige Polyurethanpolymere können beispielsweise durch Reaktion von zwei- oder mehrwertigen Alkoholen mit Diisocyanaten erhalten werden.

Besonders bevorzugte werden Polyurethane aus der Gruppe der anionischen Polyurethanpolymere, umfassend mindestens eine Struktureinheit der Formel (I) und mindestens eine Struktureinheit der Formel (II) und mindestens eine Struktureinheit der Formel (III) und mindestens eine Struktureinheit der Formel (IV) und mindestens eine Struktureinheit der Formel (V): 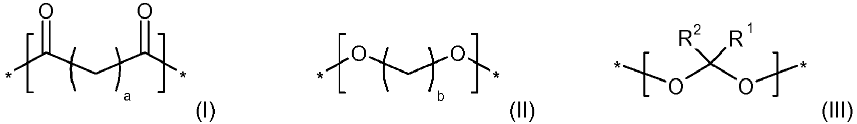 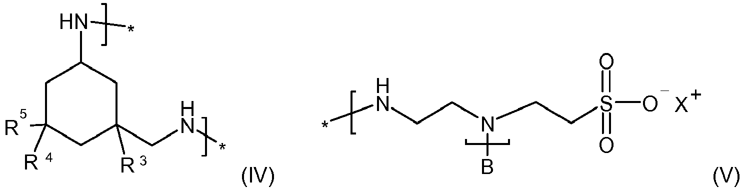 worin
- a und b, unabhängig voneinander, für ganze Zahlen von 1 bis 20 stehen,
- R¹, R², R³, R⁴ und R⁵, jeweils unabhängig voneinander, für ein Wasserstoffatom oder für eine C₁-C₄-Alkylgruppe stehen,
- B für ein Wasserstoffatom oder eine direkte Bindung zu einer weiteren Struktureinheit steht,
- X⁺ für ein physiologisch verträgliches Kation steht.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol "*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Darüber hinaus werden erfindungsgemäß unter dem Begriff "anionische Polyurethanpolymere" solche Polyurethanpolymere verstanden, welche in einem protischen Lösemittel bei Standardbedingungen mindestens eine Struktureinheit mit permanent anionischen Gruppen tragen, wobei die anionischen Gruppen durch Gegenionen unter Erhaltung der Elektroneutralität kompensiert werden müssen. Unter anionische Gruppen fallen erfindungsgemäß insbesondere Carboxyl- und Sulfonsäuregruppen.

Die besonders bevorzugten Polyurethanpolymere basieren auf Struktureinheiten der Formeln (I) bis (V). In den Struktureinheiten der Formeln (III) und (IV) können die Reste R¹ bis R⁵ für (C₁ bis C₄)-Alkylgruppen stehen. Beispiele hierfür sind Methyl-, Ethyl-, Propyl-, Isopropyl-, Butyl-, Sec-Butyl-, Isobutyl- sowie tert-Butylgruppen.

Die anionischen Polyurethanpolymere können durch Umsetzung eines isocyanatgruppenhaltigen Prepolymers (V1) mit mindestens einer aminogruppenhaltigen Verbindung (V2) erhalten werden.

Bevorzugt werden hierfür isocyanatgruppenhaltige Prepolymere (V1) verwendet, welche endständige Isocyanatgruppen aufweisen, d. h. derartige Polymere (V1) weisen Isocyanatgruppen an den Kettenenden, bevorzugt an sämtlichen Kettenenden, des Polymers auf. Weiterhin ist es bevorzugt, wenn isocyanatgruppenhaltige Prepolymere verwendet werden, welche durch Umsetzung von Isocyanaten bzw. Polyisocyanaten mit einem oder mehreren Polyolen, ausgewählt aus der Gruppe von Polyether-Polyolen, Polycarbonatpolyolen, Polyether-Polycarbonat-Polyolen und/oder Polyesterpolyolen, erhalten werden. Geeignete Prepolymere (V1) sind daher insbesondere die dem Fachmann an sich bekannten aliphatischen, aromatischen oder cycloaliphatischen Polyisocyanate mit einer NCO- Funktionalität von größer oder gleich 2.

Besonders bevorzugte Isocyanate sind Hexamethylendiisocyanat, Isophorondiisocyanat oder die isomeren Bis-(4,4'-isocyanatocyclohexyl)methane sowie Mischungen der vorgenannten Diisocyanate. Besonders bevorzugte aliphatische Polyesterpolyole sind Polyesterpolyole auf Basis aliphatischer Carbonsäuren und aliphatischer Polyole, insbesondere auf Basis von Adipinsäure und aliphatischen Alkoholen, wie Hexandiol und/oder Neopentylglykol mit einem mittleren Molekulargewicht Mw von 600 bis 3000 g/mol. Weiterhin werden bevorzugt Polycarbonate, insbesondere Polydicarbonatdiole, mit mindestens einer Hydroxylgruppe und einem mittleren Molekulargewicht Mw von 600 bis 3000 g/mol eingesetzt, welche beispielsweise durch Reaktion von Kohlensäurederivaten, wie Diphenylcarbonat, Dimethylcarbonat oder Phosgen, mit Polyolen, bevorzugt Diolen, erhältlich sind. Ebenfalls bevorzugt können Polyetherpolyole, wie Polytetramethylenglykolpolyether und Polycarbonat-Polyole bzw. deren Mischungen eingesetzt werden. Die Bestimmung des mittleren Molekulargewichts M_{w} kann beispielsweise mittels Gelpermeationschromatographie (GPC) mit Polystyrol als internem Standard, wie in der deutschen Offenlegungsschrift DE1999614603 beschrieben, erfolgen.

Die zur Herstellung der erfindungsgemäß bevorzugt eingesetzten anionischen Polyurethanpolymere verwendeten aminogruppenhaltigen Verbindungen (V2) werden vorzugsweise aus primären und/oder sekundären Aminen und/oder Diaminen ausgewählt. Insbesondere umfassen die aminogruppenhaltigen Verbindungen (V2) mindestens ein Diamin. Die aminogruppenhaltigen Verbindungen (V2) werden bevorzugt aus aminogruppenhaltigen Verbindungen, welche ionische oder ionogene (ionenbildende) Gruppen aufweisen, und aminogruppenhaltigen Verbindungen, welche keine ionischen oder ionogenen Gruppen aufweisen, ausgewählt. Geeignete aminogruppenhaltige Verbindungen (V2), welche keine ionischen oder ionogenen Gruppen aufweisen, sind bevorzugt ausgewählt aus 1,2-Ethylendiamin, Bis(4-aminocyclohexyl)methan, 1,4-Diaminobutan, Isophorondiamin, Ethanolamin, Diethanolamin und Diethylentriamin. Aminogruppenhaltige Verbindungen (V2), welche ionische und/oder ionogene Gruppen enthalten, weisen bevorzugt Sulfonat- bzw. Sulfonsäuregruppen, besonders bevorzugt Natriumsulfonatgruppen, auf. Aminogruppenhaltige Verbindungen (V2), welche ionische oder ionogene Gruppen aufweisen, sind bevorzugt ausgewählt aus Salzen von 2-(2-Aminoethylamino)ethansulfonsäure, Ethylendiaminpropyl- oder -butylsulfonsäure, 1,2- oder 1,3-Propylendiamin-β-ethylsulfonsäure sowie Taurin.

Gemäß einer ersten Ausführungsform der vorliegenden Erfindung steht in der Struktureinheit der Formel (I) a für ganze Zahlen von 1 bis 10, vorzugsweise von 2 bis 8, insbesondere von 2 bis 6 und steht in der Struktureinheit der Formel (II) b für ganze Zahlen von 1 bis 12, vorzugsweise von 2 bis 10, insbesondere von 4 bis 8.

Im Rahmen der vorliegenden Erfindung kann es darüber hinaus vorgesehen sein, dass in den Struktureinheiten der Formeln (III) und (IV) die Reste R¹, R², R³, R⁴ und R⁵, jeweils unabhängig voneinander, für Wasserstoff oder für eine Methylgruppe, insbesondere für eine Methylgruppe, stehen.

Es ist erfindungsgemäß bevorzugt, wenn in der Struktureinheit der Formel (I) X⁺ für Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom steht. Kationische organische Verbindungen können beispielsweise durch Protonierung primärer, sekundärer oder tertiärer organischer Amine mit einer Säure oder durch permanente Quaternisierung besagter organischer Amine erhalten werden. Beispiele für im Rahmen der vorliegenden Erfindung geeignete kationische organische Ammoniumverbindungen sind beispielsweise 2-Ammonioethanol und 2-Trimethylammonioethanol.

Gemäß einer besonders bevorzugten Ausführungsform der vorliegenden Erfindung enthält die erfindungsgemäße kosmetische Zubereitung a) mindestens ein anionisches Polyurethanpolymer a1), welches mindestens eine Struktureinheit der Formel (Ia) und mindestens eine Struktureinheit der Formel (IIa) und mindestens eine Struktureinheit der Formel (IIIa) und mindestens eine Struktureinheit der Formel (IVa) und mindestens eine Struktureinheit der Formel (Va) aufweist 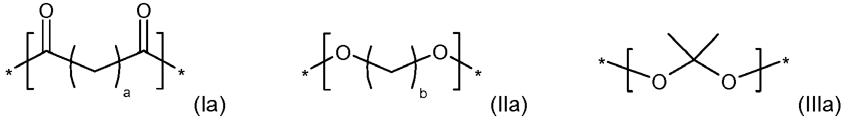 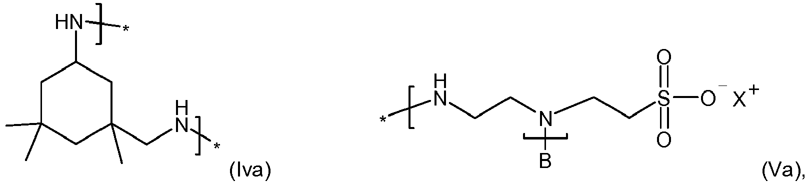 worin
a für ganze Zahlen von 2 bis 6 steht,
b für ganze Zahle von 4 bis 8 steht, und
X⁺ für ein physiologisch verträgliches Kation, insbesondere Natrium, steht. Der Einsatz der zuvor genannten speziellen anionischen Polyurethanpolymere in den erfindungsgemäßen kosmetischen Mitteln führt zu einem hervorragenden Frisurenhalt, insbesondere zu einer hervorragenden Glättungswirkung. Weiterhin wird der hervorragende Frisurenhalt über einen langen Zeitraum gewährleistet.

Ein im Rahmen dieser Ausführungsform ganz besonders bevorzugtes anionisches Polyurethanpolymer ist das unter der INCI-Bezeichnung Polyurethan-48 bekanntes Polymer. Dieses Polymer enthält Adipinsäure (a in Struktureinheit der Formel (la) steht für die ganze Zahl 4) und 1,6-Hexandiol (b gemäß Struktureinheit der Formel (IIa) steht für die ganze Zahl 6).

Erfindungsgemäß bevorzugt eingesetzte anionische Polyurethanpolymere weisen eine bestimmte Glasübergangstemperatur T_{g} auf. Daher ist es im Rahmen der vorliegenden Erfindung bevorzugt, wenn das mindestens eine anionische Polyurethanpolymer a) eine Glasübergangstemperatur T_{g} von -70 °C bis -20 °C, vorzugsweise von -65 °C bis -25 °C, bevorzugt von -60 °C bis -30 °C, insbesondere von -50 °C bis -40 °C, aufweist. Die Glasübergangstemperaturen werden mittels dynamischer Differenzkalorimetrie (DSC) bei einer Heizrate von 10 K/min, einer Starttemperatur von mindestens 30 °C unterhalb der Glasübergangstemperatur und einer Endtemperatur von mindestens 30 °C oberhalb der Glasübergangstemperatur bestimmt. Die Glasübergangstemperatur T_{g} ergibt sich als Mittelpunkttemperatur nach der Tangentenmethode.

Die erfindungsgemäßen kosmetischen Produkte umfassen neben der kosmetischen Zubereitung a) weiterhin eine Vorrichtung zur Entspannungsverdampfung. Der Ausdruck "Entspannungsverdampfung" bezeichnet im Rahmen der vorliegenden Anmeldung das Entstehen von Dampf bei Absenken des Druckes in einem mit Flüssigkeit befüllten, unter Überdruck (zur Umgebung) stehenden geschlossenen Raum. Ein entsprechender Überdruck lässt sich beispielsweise erzeugen, indem eine Menge der kosmetischen Zubereitung a) in einem abgeschlossenen Raum auf eine Temperatur T₁ erhitzt wird. In dem geschlossenen Raum hat die Flüssigkeit bei gegebener Temperatur T₁ einen Druck Sättigungsdruck p₁. Wird der geschlossenen Raum beispielsweise mittels eines Ventils zu einem nicht unter Überdruck stehenden Relaxationsraum mit dem Druck p₀ < p₁ geöffnet, so sinkt der Druck in dem zuvor geschlossenen Raum ab und im Rahmen der Ausbreitung des neuen Druckniveaus verdampft die kosmetische Zubereitung a), bzw. das in der kosmetischen Zubereitung enthaltene Lösungsmittel oder Teile dieses Lösungsmittels. Der entstehende Dampf oder Sprühnebel kann für die Applikation spezifischer kosmetischer Zubereitungen genutzt werden.

Wird die kosmetische Zubereitung a) also ausgehend von Standardbedingungen (T₀ = 25°C, p₀ = 1,000 bar) in einem geschlossenen Raum erhitzt, so resultiert neben einer erhöhten Temperatur weiterhin ein erhöhter Druck der kosmetischen Zubereitung a). Dieser erhöhte Druck kann in einem Relaxationsraum auf einen Druck p₀, zum Beispiel den umgebenden Luftdruck (p₀ = 1,000 bar), entlastet werden kann, wodurch mindestens teilweise eine Verdampfung der kosmetischen Zubereitung a) erreicht wird.

Die kosmetische Zubereitung a) kann unmittelbar in dem Raum entspannt werden, in welchem sie zuvor erhitzt wurde. Die erhitzte und unter Überdruck befindliche kosmetische Zubereitung a) kann alternativ aber auch nach dem Erhitzen in einen zweiten Raum transportiert werden, in welchem dann nachfolgend die Druckentlastung erfolgt.

Bei der Entspannungsverdampfung handelt es sich mit anderen Worten um ein Verfahren, bei welchem die kosmetische Zubereitung a) in einem geschlossenen Behälter mittels einer Heizvorrichtung auf Temperaturen oberhalb der Umgebungstemperatur erhitzt wird, wobei in dem Behälter ein Druck oberhalb des Umgebungsdrucks entsteht, und die erhitzte und unter erhöhtem Druck stehende kosmetische Zubereitung a) anschließend aus dem Behälter in die Umgebung entspannt wird.

Bei einer Vorrichtung zur Entspannungsverdampfung handelt es sich demnach um eine Vorrichtung, welche einen Behälter und eine Heizvorrichtung umfasst und derart ausgestaltet ist, dass eine kosmetische Zubereitung a) in dem geschlossenen Behälter mittels der Heizvorrichtung auf Temperaturen oberhalb der Umgebungstemperatur in einer Weise erhitzt werden kann, dass in dem Behälter ein Druck oberhalb des Umgebungsdrucks entsteht und die erhitzte und unter erhöhtem Druck stehende kosmetische Zubereitung a) aus dem Behälter in die Umgebung entspannt werden kann.

Gleichzeitig mit oder nach der Druckentlastung kann die kosmetische Zubereitung a) einer Düse zugeführt werden, mittels derer beispielsweise Eigenschaften des durch die Entspannungsverdampfung erzeugten Dampfes bzw. Sprühnebels, insbesondere die Tröpfchengröße oder die Tröpfchendichte aber auch die Sprühweite und die Form des Sprühkegels beeinflusst werden können. Der Einsatz von Düsen, vorzugsweise Zerstäuberdüsen, ist daher bevorzugt. Der spezifische Düsentyp oder die spezifische Düsengestaltung wird in Abhängigkeit von den jeweiligen Sprühnebeleigenschaften gezielt festgelegt.

Zusammenfassend verfügt eine bevorzugte Vorrichtung zur Entspannungsverdampfung über
b1) einen mittels eines Ventils zu verschließenden und zu öffnenden Behälter b1), welcher den geschlossenen Innenraum definiert, in dem die kosmetische Zubereitung aufgenommen werden kann,
b2) eine Heizvorrichtung b2), welche es ermöglicht eine in dem Behälter b1) befindliche kosmetische Zubereitung zu erhitzen.
Besonders bevorzugt ist der Einsatz einer zusätzlichen Düse b3), welche eine Zerstäubung der aus dem Behälter entweichenden kosmetischen Zubereitung a) ermöglicht. Alternativ zu einem Ventil kann auch ein vergleichbar wirkendes Schließelement, welches eine zugehörige Öffnung im Behälter durch entsprechende Positionsänderung verschließen oder freigeben kann, zum Einsatz kommen Ein bevorzugter Gegenstand der vorliegenden Erfindung ist ein kosmetisches Produkt, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer;
   wobei der Gewichtsanteil der Komponenten a) und b) am Gesamtgewicht der kosmetischen Zubereitung a) mehr als 92% beträgt
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   die kosmetische Zubereitung a) in dem geschlossenen Innenraum des Behälters b1) mittels der Heizvorrichtung b2) unter Druckerhöhung erhitzt werden kann.

Ein besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer,
   wobei der Gewichtsanteil der Komponenten a) und b) am Gesamtgewicht der kosmetischen Zubereitung a) mehr als 92% beträgt;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht ein in dem Behälter b1) befindliche kosmetische Zubereitung zu erhitzen
   b3) eine Düse b3), welche eine Zerstäubung der kosmetischen Zubereitung a) ermöglicht.

Ein besonders bevorzugter Gegenstand der vorliegenden Erfindung ist mit anderen Worten ein kosmetisches Produkt, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer,
   wobei der Gewichtsanteil der Komponenten a) und b) am Gesamtgewicht der kosmetischen Zubereitung a) mehr als 92% beträgt;
b) eine Vorrichtung zur Entspannungsverdampfung der kosmetischen Zubereitung a), wobei die Vorrichtung zur Entspannungsverdampfung einen Behälter b1) und eine Heizvorrichtung b2 umfasst und derart ausgestaltet ist, dass
   - die kosmetische Zubereitung a) in den Innenraum des Behälters b1) aufgenommen werden kann,
   - der mit der kosmetischen Zubereitung a) wenigstens anteilsweise befüllte Innenraum des Behälters b1) verschlossen werden kann,
   - die kosmetische Zubereitung a) in dem geschlossenen Innenraum des Behälters b1) mittels der Heizvorrichtung b2) unter Druckerhöhung erhitzt werden kann,
   - die erhitzte kosmetische Zubereitung a) aus dem Innenraum des Behälters b1) unter Druckminderung in die Umgebung entspannt werden kann.

Der Behälter b1), in welchem die kosmetische Zubereitung erhitzt wird, ist in einer Weise ausgestaltet, die es ermöglicht, diesen Behälter während der Erhitzung der kosmetischen Zubereitung a) gegen die Umgebung vollständig abzuschließen und nach der Erhitzung, zur Ermöglichung Entspannungsverdampfung der kosmetischen Zubereitung a), zu öffnen. Dies kann beispielsweise durch ein Bauteil zur Durchflussregelung, insbesondere ein Ventil, gewährleistet werden.

Der Behälter b1), in welchem die kosmetische Zubereitung erhitzt wird, steht vorzugsweise in Kontakt mit einem weiteren Behälter, aus welchem die zur Entspannungsverdampfung vorgesehene Menge der kosmetischen Zubereitung vor der Erhitzung in den Behälter b1) überführt wird. Der Zugang zwischen diesem Vorratsbehälter und Behälter b1) ist dabei über eine entsprechende Vorrichtung, beispielsweise ein Ventil, zu öffnen und zu schließen. Dieser weitere Behälter ist vorzugsweise in Form eines Vorratsbehälters ausgestaltet, das heißt, er umfasst bevorzugt ein Vielfaches, beispielsweise mehr als das Zehnfache, vorzugsweise mehr als das Fünfzigfache, der für einen Verdampfungsvorgang notwendigen Menge der kosmetischen Zubereitung. Mit anderen Worten weist der weitere Behälter/Vorratsbehälter vorzugsweise ein Vielfaches, beispielsweise mehr als das Zehnfache Volumen, vorzugsweise mehr als das Zwanzigfache und insbesondere mehr als das Fünfzigfache Volumen des Behälters b1) auf.

Ein weiterer besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer,
   wobei der Gewichtsanteil der Komponenten a) und b) am Gesamtgewicht der kosmetischen Zubereitung a) mehr als 92% beträgt;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Zwanzigfache und insbesondere mindestens das Fünfzigfache Volumen des Behälters b1) aufweist.

Der Vorratsbehälter ist kein Druckbehälter und die in dem Vorratsbehälter befindliche kosmetische Zusammensetzung befindet sich nicht unter Druck, mit anderen Worten entspricht der Druck im Inneren des Vorratsbehälters dem Umgebungsdruck (auch Luftdruck oder Atmosphärendruck). So umfassen entsprechende kosmetische Produkte beispielsweise keine Treibmittel. Auch verfügt das kosmetische Produkt über keine Pumpvorrichtung, die geeignet ist, die kosmetische Zubereitung ohne die Einwirkung der Vorrichtung zur Entspannungsverdampfung in die Umgebung freizusetzen oder zu versprühen.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer,
   wobei der Gewichtsanteil der Komponenten a) und b) am Gesamtgewicht der kosmetischen Zubereitung a) mehr als 92% beträgt;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht.

Ein ganz besonders bevorzugter Gegenstand der vorliegenden Erfindung ist daher ein kosmetisches Produkt, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer,
   wobei der Gewichtsanteil der Komponenten a) und b) am Gesamtgewicht der kosmetischen Zubereitung a) mehr als 92% beträgt;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst.

Bevorzugt werden weiterhin kosmetische Produkte, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer,
   wobei der Gewichtsanteil der Komponenten a) und b) am Gesamtgewicht der kosmetischen Zubereitung a) mehr als 92% beträgt;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht,
   wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Zusammenfassend ist ein besonders bevorzugter Gegenstand der vorliegenden Erfindung daher ein kosmetisches Produkt, umfassend
a) eine kosmetische Zubereitung, enthaltend bezogen auf ihr Gesamtgewicht
   a1) mindestens 88 Gew.-% polares Lösungsmittel;
   a2) mindestens 0,1 Gew.-% filmbildendes Polymer,
   wobei der Gewichtsanteil der Komponenten a) und b) am Gesamtgewicht der kosmetischen Zubereitung a) mehr als 92% beträgt;
b) eine Vorrichtung zur Entspannungsverdampfung der kosmetischen Zubereitung a), umfassend
   b1) einen mittels eines Ventils zu verschließenden und zu öffnenden Behälter b1)
   b2) eine Heizvorrichtung, welche es ermöglicht eine in dem geschlossenen Behälter b1) befindliche kosmetische Zubereitung zu erhitzen
   b3) eine Düse b3), welche eine Zerstäubung der kosmetischen Zubereitung a) ermöglicht.
c) einen Vorratsbehälter für die kosmetische Zubereitung a), aus welchem die kosmetische Zubereitung a) in den Behälter b1) gelangen kann, wobei
   ∘ der Zugang zwischen Vorratsbehälter und Behälter b1) ein Bauteil zur Durchflussregelung aufweist, mittels dessen der Durchfluss der kosmetischen Zubereitung a) aus dem Vorratsbehälter in den Behälter b1) unterbrochen werden kann;
   ∘ der Vorratsbehälter mindestens das Zehnfache Volumen, vorzugsweise mindestens das Fünfzigfache Volumen des Behälters b1) aufweist;
   ∘ der Druck im Inneren des Vorratsbehälters dem Umgebungsdruck entspricht und das kosmetische Produkt kein Treibmittel umfasst,
   wobei das kosmetische Produkt keine Pumpvorrichtung aufweist, die geeignet ist, die kosmetische Zubereitung a) ohne die Einwirkung der Vorrichtung zur Entspannungsverdampfung freizusetzen oder zu versprühen.

Als weitere geeignete Wirk- oder Hilfsstoffe sind insbesondere zusätzliche Pflegestoffe zu nennen.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Panthenol, Coffein, Nicotinamid und Sorbitol.

Als Pflegestoff können die erfindungsgemäßen Mittel weiterhin mindestens einen Pflanzenextrakt, aber auch Mono- bzw. Oligosaccharide und/oder Lipide enthalten.

Neben den beiden zuvor beschriebenen Bestandteilen a1) und a2) können die erfindungsgemäßen kosmetischen Zubereitungen a) weitere Wirk- oder Hilfsstoffe enthalten, wobei insbesondere solche Wirk- oder Hilfsstoffe bevorzugt werden, welche die Herstellbarkeit, Applizierbarkeit und/oder kosmetische Wirkung erfindungsgemäßer kosmetischer Zubereitungen verbessern.

Ein erstes Beispiel für einen bevorzugten Wirk- und Hilfsstoff sind die kationischen Tenside a3). Bevorzugte kationische Tenside a3) sind ausgewählt unter quartären Ammoniumverbindungen, Esterquats und Amidoaminen. Die kationischen Tenside sind in der kosmetischen Zubereitung a), bezogen auf deren Gesamtgewicht, in Mengen von 0,01 bis 4,0 Gew.-%, vorzugsweise 0,05 bis 2,0 Gew.-%, und insbesondere 0,1 bis 1,0 Gew.-% enthalten. Kationsiches Tenside a3) aus der Gruppe der quartären Ammoniumverbindungen sind besonders bevorzugt.

Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Ganz besonders bevorzugte erfindungsgemäße kosmetische Zubereitungen a) sind dadurch gekennzeichnet, dass sie bezogen auf ihr Gesamtgewicht 0,01 bis 4,0 Gew.-%, vorzugsweise 0,05 bis 2,0 Gew.-%, und insbesondere 0,1 bis 1,0 Gew.-% (C₁₂ bis C₁₈)-Alkyltrimethylammoniumsalz(e) enthalten.

Ein zweiter besonders bevorzugter Inhaltsstoff der kosmetischen Zubereitungen a) sind die Proteinhydrolysate a4). Diese gegebenenfalls derivatisierten Substanzen können natürlichen oder synthetischen Ursprung sein. Der Gewichtanteil des Proteinhydrolysats a4) am Gesamtgewicht der kosmetischen Zubereitung a) beträgt vorzugsweise 0,01 bis 2,0 Gew.-%, bevorzugt 0,02 bis 1,0 Gew.-%, und insbesondere 0,05 bis 0,8 Gew.-%.

Die Gruppe der besonders bevorzugten natürlichen Proteinhydrolysate, also der Hydrolysate natürlicher Proteine, lässt sich in Proteinhydrolysate pflanzlichen und tierischen Ursprungs unterteilen.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden u.a. unter den Handelsnamen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.
Pflanzliche Proteinhydrolysate sind beispielsweise Soja-, Mandel-, Erbsen-, Kartoffel- und Weizenproteinhydrolysate. Solche Produkte sind u.a. unter den Handelsnamen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Die Gruppe der marinen Proteinhydrolysate umfasst Proteinhydrolysate tierischen und pflanzlichen Ursprunges. Zu den marinen Proteinhydrolysaten zählen beispielsweise Kollagenhydrolysate von Fischen oder Algen, Proteinhydrolysate von Muscheln und die Perlenhydrolysate. Beispiele für erfindungsgemäße Perlenhydrolysate oder Perlenextrakte sind die unter den Handelsnamen Pearl Protein Extract BG® oder Crodarom® Pearl vertriebenen Substanzgemische.

Besonders bevorzugt sind Proteinhydrolysate auf der Basis von Kollagen, Seidenprotein, Keratin, Milchprotein, Sojaprotein, Mandelprotein, Weizenprotein oder Perlen. Höchst bevorzugt ist der Einsatzder Hydrolysate von Keratin, Seidenprotein und Weizenprotein.

Die Zusammensetzung einiger besonders bevorzugter erfindungsgemäßer kosmetischer Zubereitungen kann den folgenden Tabellen entnommen werden (Angaben in Gew.-% bezogen auf das Gesamtgewicht des kosmetischen Mittels sofern nicht anders angegeben). Bezüglich weiterer bevorzugter Ausführungsformen dieser besonders bevorzugten Zusammensetzungen gilt mutatis mutandis das zuvor zu den erfindungsgemäßen kosmetischen Zubereitungen a) Gesagte.

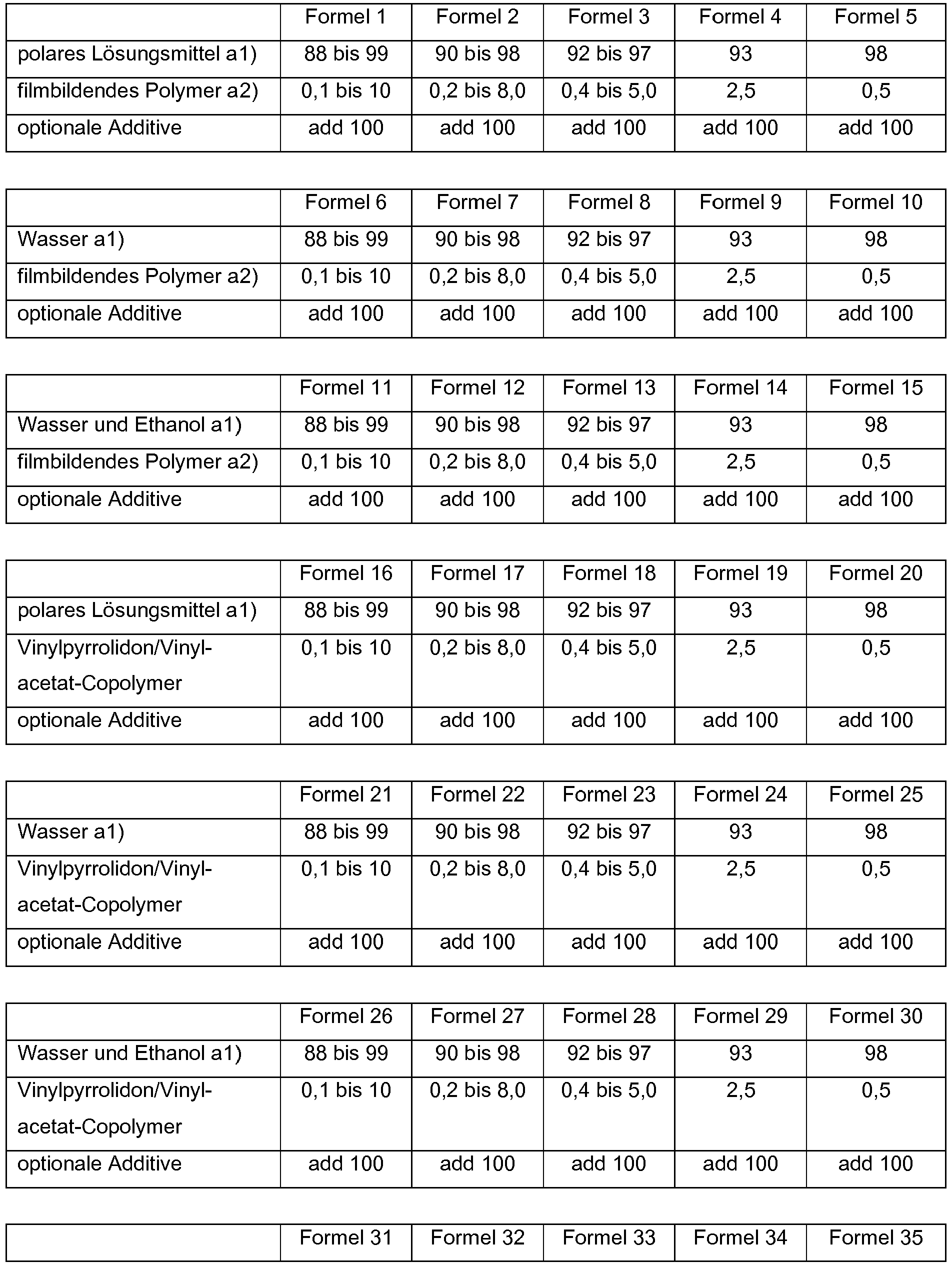

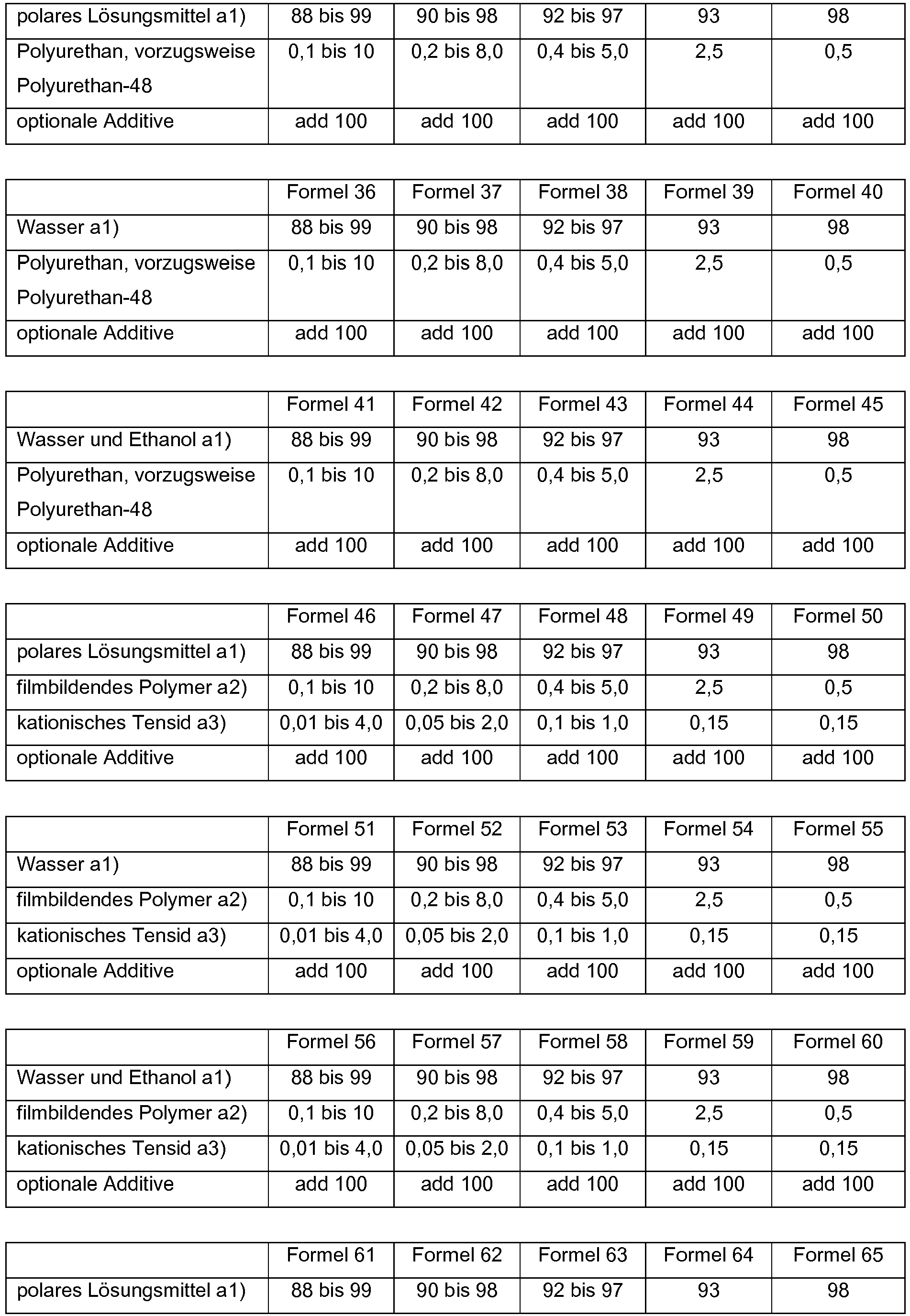

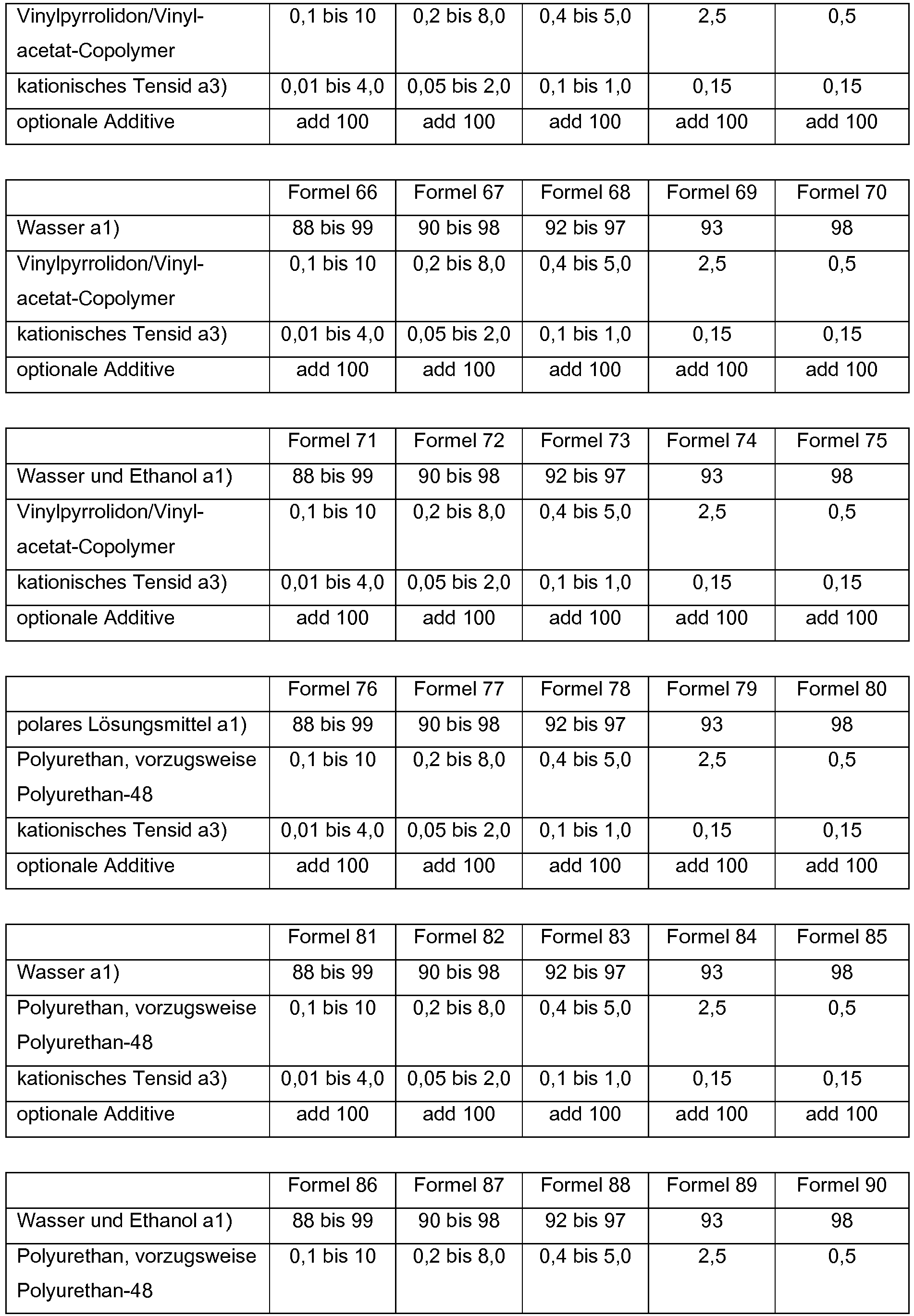

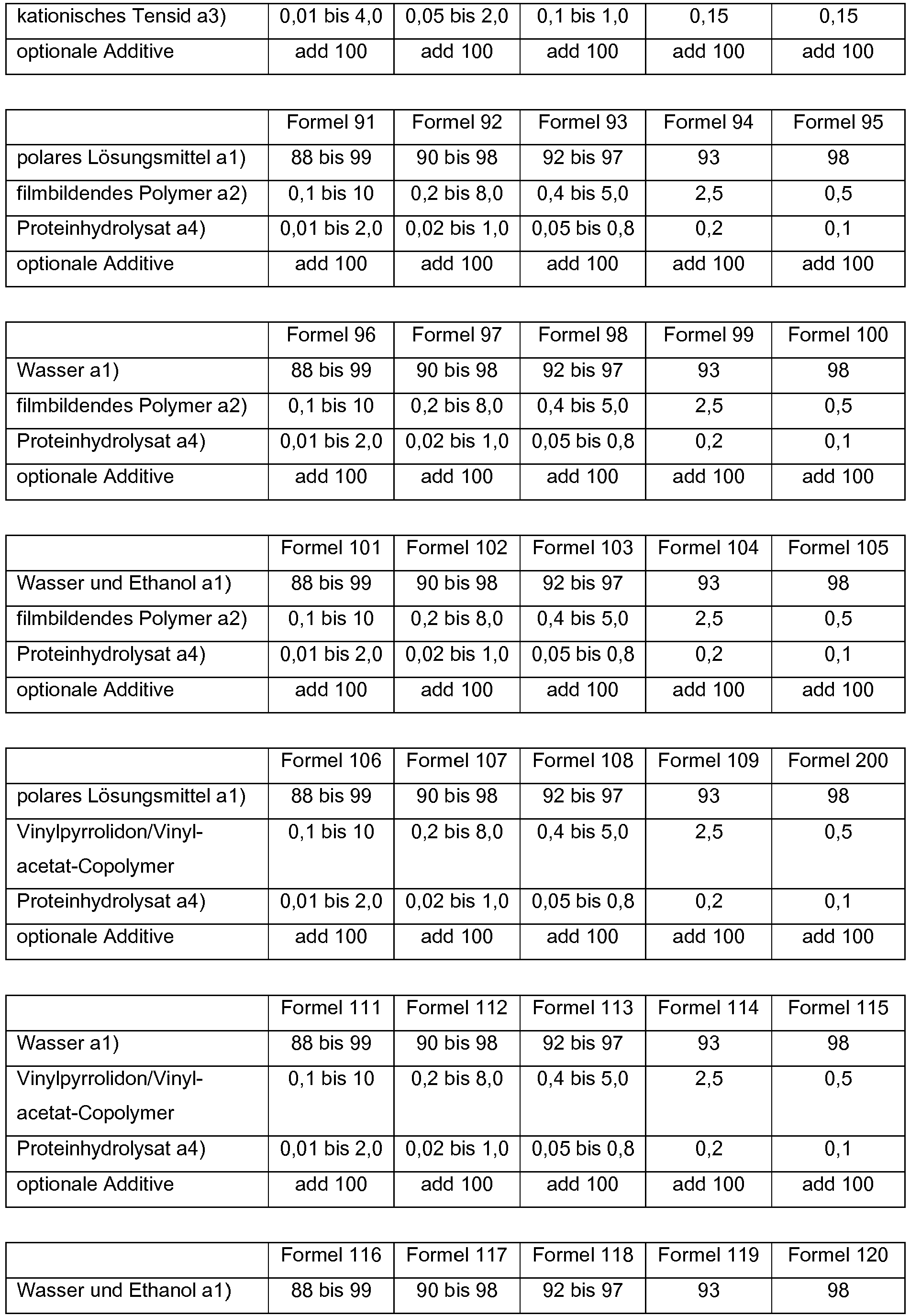

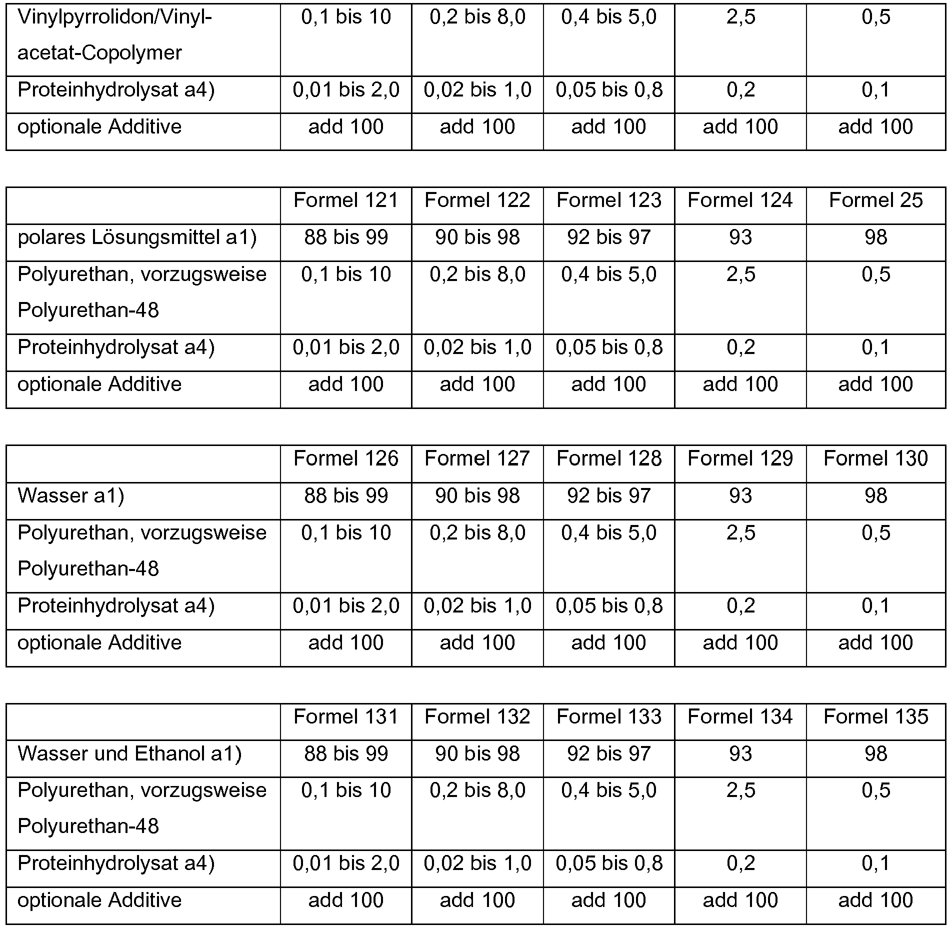

Ganz besonders bevorzugte kosmetische Zubereitungen enthalten neben den zuvor beschriebenen Bestandteilen a1) bis a4) nur geringe Mengen weiterer Wirk- und Hilfsstoffe. Kosmetische Zubereitungen, dadurch gekennzeichnet, dass der Gewichtsanteil der Bestandteile a1), a2) sowie, falls enthalten, a3) und/oder a4) am Gesamtgewicht der kosmetischen Zubereitung mindestens 93 Gew.-%, vorzugsweise mindestens 95 Gew.-% und insbesondere mindestens 97 Gew.-% beträgt, sind aufgrund ihrer einfachen Herstellbarkeit und guten kosmetischen Wirkung besonders bevorzugt. Ganz besonders bevorzugte kosmetische Zubereitungen bestehen, bezogen auf ihr Gesamtgewicht, zu mindestens 94 Gew.-%, vorzugsweise mindestens 96 Gew.-% aus den Bestanteilen a1) und a2).

Wie eingangs ausgeführt eignen sich die erfindungsgemäßen kosmetischen Zubereitungen a) in besonderer Weise für die Applikation mittels einer Vorrichtung zur Entspannungsverdampfung. Ein weiterer Gegenstand der vorliegenden Anmeldung ist daher die Verwendung einer kosmetischen Zubereitung a) enthaltend, bezogen auf ihr Gesamtgewicht,
a1) mindestens 88 Gew.-% polares Lösungsmittel;
a2) mindestens 0,1 Gew.-% filmbildendes Polymer,
wobei der Gewichtsanteil der Komponenten a1) und a2) am Gesamtgewicht der kosmetischen Zubereitung a) mehr als 92% beträgt, als Prozessgut in einer Vorrichtung zur Entspannungsverdampfung.

Gegenstand der vorliegenden Anmeldung ist zudem die Verwendung eines erfindungsgemäßen Produkts zur Beaufschlagung keratinhaltiger Fasern, insbesondere menschlicher Haare, mit einer kosmetischen Zubereitung a) bzw. zur temporären Verformung keratinhaltiger Fasern, insbesondere menschlicher Haare.

Ein Verfahren zur temporären Verformung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) mindestens 88 Gew.-% polares Lösungsmittel;
a2) mindestens 0,1 Gew.-% filmbildendes Polymer,
wobei der Gewichtsanteil der Komponenten a1) und a2) am Gesamtgewicht der kosmetischen Zubereitung a) mehr als 92% beträgt, beaufschlagt werden, ist ein weiterer Gegenstand der vorliegenden Anmeldung. Die kosmetische Zubereitung a) wird mittels der Vorrichtung zur Entspannungsverdampfung vorzugsweise in einen Sprühnebel überführt, welcher nachfolgend die keratinhaltigen Fasern beaufschlagt.

Die erfindungsgemäßen Mittel haben besondere Vorteile im Rahmen durch Hitze herbeigeführter Haarumformungen. Ein Verfahren zur temporären Verformung keratinhaltiger Fasern, vorzugsweise menschlicher Haare, in dessen Verlauf die keratinhaltigen Fasern einer Wärmebehandlung mit Temperaturen von 50°C bis 350°C, vorzugsweise von 80 bis 300°C, besonders bevorzugt von 120 bis 260 °C und insbesondere von 150 bis 220°C unterworfen werden, dadurch gekennzeichnet, dass die keratinhaltigen Fasern vor der Wärmebehandlung mithilfe eines erfindungsgemäßen kosmetischen Produkts mit einer kosmetischen Zubereitung a) beaufschlagt werden, ist daher erfindungsgemäß besonders bevorzugt.

Ein weiterer Anmeldungsgegenstand ist die Verwendung eines erfindungsgemäßen kosmetischen Produkts zur Erzielung einer Hitzeschutzwirkung im Rahmen von Haarumformungsverfahren, bei denen die keratinhaltigen Fasern einer Wärmebehandlung mit Temperaturen von 50°C bis 350°C, vorzugsweise von 80 bis 300°C, besonders bevorzugt von 120 bis 260 °C und insbesondere von 150 bis 220°C unterworfen werden.

Um eine ausreichende Sprühwirkung zu erzielen, wird die kosmetische Zubereitung a) vorzugsweise auf Temperaturen oberhalb des Siedepunktes des in der kosmetischen Zubereitung a) enthaltenen polaren Lösungsmittels oder Lösungsmittelgemisches erhitzt.

Handelt es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches), wird die kosmetische Zubereitung vorzugsweise auf Temperaturen oberhalb 100°C, vorzugsweise auf Temperaturen von 100°C und 240°C, besonders bevorzugt auf Temperaturen von 140°C bis 160°C erhitzt.

Der durch die Erhitzung der kosmetischen Zubereitung a) erzielte Überdruck beträgt in den Fällen, in denen es sich bei dem polaren Lösungsmittel um Wasser oder Lösungsmittelgemische mit einem Wasseranteil oberhalb 50 Gew.-% (bezogen auf das Gesamtgewicht des Lösungsmittelgemisches) handelt, vorzugsweise zwischen 1,1 und 8 bar, bevorzugt zwischen 1,2 und 4 bar.

Ein bevorzugter Anmeldungsgegenstand ist ein Verfahren zur Farbveränderung keratinhaltiger Fasern, insbesondere menschlicher Haare, bei welchem die keratinhaltigen Fasern mittels einer Vorrichtung zur Entspannungsverdampfung mit einer kosmetischen Zubereitung a) enthaltend bezogen auf ihr Gesamtgewicht
a1) mindestens 88 Gew.-% polares Lösungsmittel;
a2) mindestens 0,1 Gew.-% filmbildendes Polymer,
wobei der Gewichtsanteil der Komponenten a1) und a2) am Gesamtgewicht der kosmetischen Zubereitung a) mehr als 92% beträgt, beaufschlagt werden, wobei
- aus einem Vorratsbehälter, in dessen Inneren ein Druck herrscht, der dem Umgebungsdruck entspricht, eine Teilmenge der in diesem Vorratsbehälter befindlichen kosmetischen Zubereitung a) in einen Behälter b1) überführt wird;
- nachfolgend der Zugang zwischen Vorratsbehälter und Behälter b1) durch ein Bauteil zur Durchflussregelung, mittels dessen der Durchfluss der kosmetischen Zubereitung a) aus dem Vorratsbehälter in den Behälter b1) unterbrochen werden kann, unterbrochen wird;
- nachfolgend die in dem gegen die Umgebung abgeschlossenen Behälter b1) befindliche kosmetische Zubereitung a) mittels einer Heizvorrichtung erhitzt wird, so dass der Druck im Inneren des Behälters b1) auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter b1) in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter b1) befindlichen kosmetischen Zubereitung aus dem Behälter b1) in die Umgebung unter Minderung des zum Zeitpunkt der Behälteröffnung in dem Behälter b1) herrschenden Drucks, entspannt wird.

Die Entspannung der kosmetischen Zubereitung a) in die Umgebung erfolgt vorzugsweise unter Ausbildung eines Sprühnebels der kosmetischen Zubereitung a).

Die aus dem Behälter b1) entspannte kosmetische Zubereitung a) wird vorzugsweise auf keratinische Fasern, insbesondere menschliche Haare aufgebracht.

Verfahren, in deren Verlauf die aus dem Behälter b1) entspannte kosmetische Zubereitung vor der Beaufschlagung der keratinischen Fasern durch eine Düse geleitet wird, sind besonders bevorzugt.

Bezüglich weiterer bevorzugter Ausführungsformen der erfindunsgemäßen Verwendungen und des erfindungsgemäßen Verfahrens gilt mutatis mutandis das zu den erfindungsgemäßen kosmetischen Zubereitungen a) und zu der Vorrichtung zur Entspannungsverdampfung b) Gesagte.

## Patentansprüche

1. Kosmetisches Produkt, umfassend
a) eine kosmetische Zubereitung, enthaltend bezogen auf ihr Gesamtgewicht
a1) mindestens 88 Gew.-% polares Lösungsmittel;
a2) mindestens 0,1 Gew.-% filmbildendes Polymer;
wobei der Gewichtsanteil der Komponenten a1) und a2) am Gesamtgewicht der kosmetischen Zubereitung a) mehr als 92% beträgt
b) eine Vorrichtung zur Entspannungsverdampfung der kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung befüllten Innenraum des Behälters zu verschließen und zu öffnen,
b3) einer Heizvorrichtung, um die in dem geschlossenen Innenraum des Behälters befindliche kosmetische Zubereitung unter Druckerhöhung zu erhitzen, sowie die erhitzte kosmetische Zubereitung aus dem Innenraum des Behälters unter Druckminderung in die Umgebung zu entspannen,
b4) einer Düse, welche eine Zerstäubung der aus dem Behälter entweichenden kosmetischen Zubereitung ermöglicht,
c) einen Vorratsbehälter für die kosmetische Zubereitung, aus welchem die kosmetische Zubereitung in den Behälter gelangen kann, wobei
- der Zugang zwischen Vorratsbehälter und Behälter ein Bauteil zur Durchflussregelung aufweist, mittels dessen der Durchfluss der kosmetischen Zubereitung aus dem Vorratsbehälter in den Behälter unterbrochen werden kann,
- der Vorratsbehälter mindestens das Zehnfache Volumen, vorzugsweise mindestens das Zwanzigfache und insbesondere mindestens das Fünfzigfache Volumen des Behälters aufweist,
- der Druck im Inneren des Vorratsbehälters dem Umgebungsdruck entspricht.

2. Kosmetisches Produkt nach Anspruch 1, **dadurch gekennzeichnet, dass** der Gewichtsanteil des polaren Lösungsmittels a1) am Gesamtgewicht der kosmetischen Zubereitung 88 bis 99 Gew.-%, vorzugsweise 90 bis 98 Gew.-% und insbesondere 92 bis 97 Gew.-% beträgt.

3. Kosmetisches Produkte nach einem der vorherigen Ansprüche, **dadurch gekennzeichnet, dass** das polare Lösungsmittel a1) ausgewählt ist aus der Gruppe Ethanol und Wasser.

4. Kosmetisches Produkt nach einem der vorherigen Ansprüche, **dadurch gekennzeichnet, dass** der Gewichtsanteil des filmbildenden Polymers a2) am Gesamtgewicht der kosmetischen Zubereitung 0,1 bis 10 Gew.-%, vorzugsweise 0,2 bis 8,0 Gew.-% und insbesondere 0,4 bis 5,0 Gew.-% beträgt.

5. Kosmetisches Produkt nach einem der vorherigen Ansprüche, **dadurch gekennzeichnet, dass** das filmbildende Polymer a2) ausgewählt ist aus der Gruppe der nichtionischen Polymere, vorzugsweise aus der Gruppe der Polyvinylpyrrolidone und Vinylpyrrolidon/Vinylacetat-Copolymere, insbesondere der Vinylpyrrolidon/Vinylacetat-Copolymere.

6. Kosmetisches Produkt nach einem der vorherigen Ansprüche, **dadurch gekennzeichnet, dass** das filmbildende Polymer a2) ausgewählt ist aus der Gruppe Polyurethane, vorzugsweise aus der Gruppe der anionischen Polyurethanpolymere, umfassend mindestens eine Struktureinheit der Formel (I) und mindestens eine Struktureinheit der Formel (II) und mindestens eine Struktureinheit der Formel (III) und mindestens eine Struktureinheit der Formel (IV) und mindestens eine Struktureinheit der Formel (V): 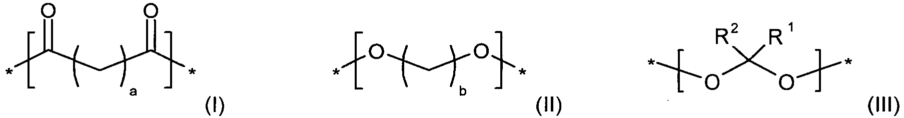 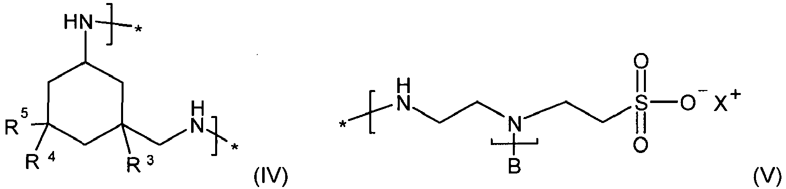 worin
- a und b, unabhängig voneinander, für ganze Zahlen von 1 bis 20 stehen,
- R¹, R², R³, R⁴ und R⁵, jeweils unabhängig voneinander, für ein Wasserstoffatom oder für eine C₁-C₄-Alkylgruppe stehen,
- B für ein Wasserstoffatom oder eine direkte Bindung zu einer weiteren Struktureinheit steht,
- X⁺ für ein physiologisch verträgliches Kation steht.

7. Verwendung einer kosmetischen Zubereitung enthaltend, bezogen auf ihr Gesamtgewicht,
a) eine kosmetische Zubereitung, enthaltend bezogen auf ihr Gesamtgewicht
a1) mindestens 88 Gew.-% polares Lösungsmittel;
a2) mindestens 0,1 Gew.-% filmbildendes Polymer,
wobei der Gewichtsanteil der Komponenten a1) und a2) am Gesamtgewicht der kosmetischen Zubereitung mehr als 92% beträgt,
als Prozessgut in einer Vorrichtung zur Entspannungsverdampfung der kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung befüllten Innenraum des Behälters zu verschließen und zu öffnen,
b3) einer Heizvorrichtung, um die in dem geschlossenen Innenraum des Behälters befindliche kosmetische Zubereitung unter Druckerhöhung zu erhitzen, sowie die erhitzte kosmetische Zubereitung aus dem Innenraum des Behälters unter Druckminderung in die Umgebung zu entspannen,
b4) einer Düse, welche eine Zerstäubung der aus dem Behälter entweichenden kosmetischen Zubereitung ermöglicht,
c) einem Vorratsbehälter für die kosmetische Zubereitung, aus welchem die kosmetische Zubereitung in den Behälter gelangen kann, wobei
- der Zugang zwischen Vorratsbehälter und Behälter ein Bauteil zur Durchflussregelung aufweist, mittels dessen der Durchfluss der kosmetischen Zubereitung aus dem Vorratsbehälter in den Behälter unterbrochen werden kann,
- der Vorratsbehälter mindestens das Zehnfache Volumen, vorzugsweise mindestens das Zwanzigfache und insbesondere mindestens das Fünfzigfache Volumen des Behälters aufweist,
- der Druck im Inneren des Vorratsbehälters dem Umgebungsdruck entspricht.

8. Verwendung eines Produkts nach einem der Ansprüche 1 bis 6 zur Beaufschlagung keratinhaltiger Fasern, insbesondere menschlicher Haare, mit einer kosmetischen Zubereitung a).

9. Verwendung eines Produkts nach einem der Ansprüche 1 bis 6 zur temporären Verformung keratinhaltiger Fasern, insbesondere menschlicher Haare.

10. Verfahren zur temporären Verformung keratinhaltiger Fasern, insbesondere menschlicher Haare, bei welchem die keratinhaltigen Fasern mittels einer Vorrichtung zur Entspannungsverdampfung einer kosmetischen Zubereitung mit
b1) einem Behälter, welcher einen geschlossenen Innenraum definiert, in dem die kosmetische Zubereitung aufgenommen werden kann,
b2) einem Ventil oder einem vergleichbar wirkenden Schließelement, um den wenigstens anteilsweise mit der kosmetischen Zubereitung befüllten Innenraum des Behälters zu verschließen und zu öffnen,
b3) einer Heizvorrichtung, um die in dem geschlossenen Innenraum des Behälters befindliche kosmetische Zubereitung unter Druckerhöhung zu erhitzen, sowie die erhitzte kosmetische Zubereitung aus dem Innenraum des Behälters unter Druckminderung in die Umgebung zu entspannen,
b4) einer Düse, welche eine Zerstäubung der aus dem Behälter entweichenden kosmetischen Zubereitung ermöglicht,
c) einem Vorratsbehälter für die kosmetische Zubereitung, aus welchem die kosmetische Zubereitung in den Behälter gelangen kann, wobei
- der Zugang zwischen Vorratsbehälter und Behälter ein Bauteil zur Durchflussregelung aufweist, mittels dessen der Durchfluss der kosmetischen Zubereitung aus dem Vorratsbehälter in den Behälter unterbrochen werden kann,
- der Vorratsbehälter mindestens das Zehnfache Volumen, vorzugsweise mindestens das Zwanzigfache und insbesondere mindestens das Fünfzigfache Volumen des Behälters aufweist,
- der Druck im Inneren des Vorratsbehälters dem Umgebungsdruck entspricht,
mit einer kosmetischen Zubereitung a) enthaltend bezogen auf ihr Gesamtgewicht
a1) mindestens 88 Gew.-% polares Lösungsmittel;
a2) mindestens 0,1 Gew.-% filmbildendes Polymer,
wobei der Gewichtsanteil der Komponenten a1) und a2) am Gesamtgewicht der kosmetischen Zubereitung mehr als 92% beträgt, beaufschlagt werden.

11. Verfahren nach Anspruch 10, **dadurch gekennzeichnet, dass**
- aus dem Vorratsbehälter eine Teilmenge der in diesem Vorratsbehälter befindlichen kosmetischen Zubereitung in den Behälter überführt wird;
- nachfolgend der Zugang zwischen Vorratsbehälter und Behälter durch ein Bauteil zur Durchflussregelung, mittels dessen der Durchfluss der kosmetischen Zubereitung aus dem Vorratsbehälter in den Behälter unterbrochen werden kann, unterbrochen wird;
- nachfolgend die in dem gegen die Umgebung abgeschlossenen Behälter befindliche kosmetische Zubereitung mittels einer Heizvorrichtung erhitzt wird, so dass der Druck im Inneren des Behälters auf Werte oberhalb des Umgebungsdrucks, vorzugsweise auf Werte zwischen 1,1 und 8 bar, insbesondere auf Werte zwischen 1,2 und 4 bar ansteigt;
- nachfolgend der unter einem Druck oberhalb des Umgebungsdrucks stehende Behälter in einer Weise geöffnet, wird, welche den Austritt mindestens einer Teilmenge, vorzugsweise mindestens 50 Gew.-%, bevorzugt mindestens 80 Gew.-% und insbesondere mindestens 90 Gew.-% der in dem Behälter befindlichen kosmetischen Zubereitung aus dem Behälter in die Umgebung unter Minderung des zum Zeitpunkt der Behälteröffnung in dem Behälter herrschenden Drucks, entspannt wird.

## Claims

1. A cosmetic product, comprising
a) a cosmetic preparation, containing, based on the total weight thereof,
a1) at least 88 wt.% polar solvent;
a2) at least 0.1 wt.% film-forming polymer;
wherein the proportion by weight of the components a1) and a2) with respect to the total weight of the cosmetic preparation a) is more than 92%
b) a device for flash evaporation of the cosmetic preparation, said device comprising
b1) a container which defines a closed interior in which the cosmetic preparation can be received,
b2) a valve or a similarly functioning closure element for closing and opening the interior of the container which is at least partly filled with the cosmetic preparation,
b3) a heating device for heating, under increased pressure, the cosmetic preparation that is located in the closed interior of the container, and releasing, under reduced pressure, the heated cosmetic preparation from the interior of the container into the surroundings,
b4) a nozzle which allows atomization of the cosmetic preparation escaping from the container,
c) a storage container for the cosmetic preparation, from which storage container the cosmetic preparation can enter the container, wherein
- the access between the storage container and the container has a component for flow control, by means of which component the flow of the cosmetic preparation from the storage container into the container can be interrupted,
- the storage container has at least ten times the volume, preferably at least twenty times and in particular at least fifty times the volume of the container, and
- the pressure inside the storage container corresponds to the ambient pressure.

2. The cosmetic product according to claim 1, **characterized in that** the proportion by weight of the polar solvent a1) with respect to the total weight of the cosmetic preparation is 88 to 99 wt.%, preferably 90 to 98 wt.% and in particular 92 to 97 wt.%.

3. The cosmetic product according to one of the preceding claims, **characterized in that** the polar solvent a1) is selected from the group consisting of ethanol and water.

4. The cosmetic product according to one of the preceding claims, **characterized in that** the proportion by weight of the film-forming polymer a2) with respect to the total weight of the cosmetic preparation is 0.1 to 10 wt.%, preferably 0.2 to 8.0 wt.% and in particular 0.4 to 5.0 wt.%.

5. The cosmetic product according to one of the preceding claims, **characterized in that** the film-forming polymer a2) is selected from the group of non-ionic polymers, preferably from the group of polyvinylpyrrolidones and vinylpyrrolidone/vinyl acetate copolymers, in particular of vinylpyrrolidone/vinyl acetate copolymers.

6. The cosmetic product according to one of the preceding claims, **characterized in that** the film-forming polymer a2) is selected from the group polyurethanes, preferably from the group of anionic polyurethane polymers, comprising at least one structural unit of formula (I) and at least one structural unit of formula (II) and at least one structural unit of formula (III) and at least one structural unit of formula (IV) and at least one structural unit of formula (V): 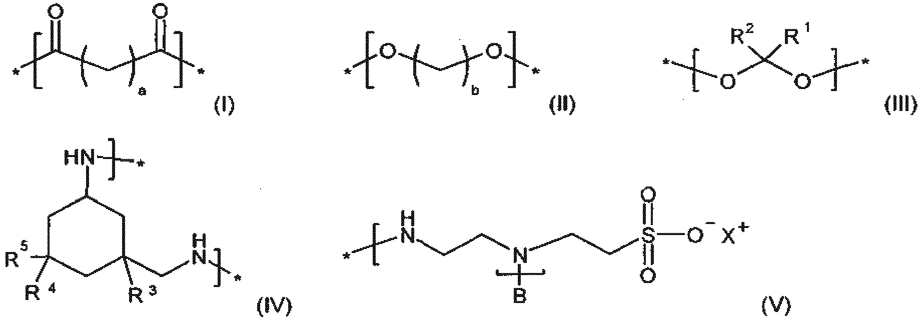 where
- a and b represent, independently of one another, integers from 1 to 20,
- R¹, R², R³, R⁴ and R⁵ represent, each independently of one another, a hydrogen atom or a C₁-C₄ alkyl group,
- B represents a hydrogen atom or a direct bond to a further structural unit, and
- X⁺ represents a physiologically acceptable cation.

7. The use of a cosmetic preparation containing, based on the total weight thereof,
a) a cosmetic preparation, containing, based on the total weight thereof,
a1) at least 88 wt.% polar solvent;
a2) at least 0.1 wt.% film-forming polymer,
wherein the proportion by weight of components a1) and a2) with respect to the total weight of the cosmetic preparation is more than 92%,
as a process material in a device for flash evaporation of the cosmetic preparation, said device comprising
b1) a container which defines a closed interior in which the cosmetic preparation can be received,
b2) a valve or a similarly functioning closure element for closing and opening the interior of the container which is at least partly filled with the cosmetic preparation,
b3) a heating device for heating, under increased pressure, the cosmetic preparation that is located in the closed interior of the container, and releasing, under reduced pressure, the heated cosmetic preparation from the interior of the container into the surroundings,
b4) a nozzle which allows atomization of the cosmetic preparation escaping from the container,
c) a storage container for the cosmetic preparation, from which the cosmetic preparation can enter the container, wherein
- the access between the storage container and the container has a component for flow control, by means of which component the flow of the cosmetic preparation from the storage container into the container can be interrupted,
- the storage container has at least ten times the volume, preferably at least twenty times and in particular at least fifty times the volume of the container, and
- the pressure inside the storage container corresponds to the ambient pressure.

8. The use of a product according to one of claims 1 to 6 for applying a cosmetic preparation a) to keratin-containing fibers, in particular human hair.

9. The use of a product according to one of claims 1 to 6 for temporarily shaping keratin-containing fibers, in particular human hair.

10. A method for temporarily shaping keratin-containing fibers, in particular human hair, in which method, by means of a device for flash evaporation of a cosmetic preparation, said device comprising
b1) a container which defines a closed interior in which the cosmetic preparation can be received,
b2) a valve or a similarly functioning closure element for closing and opening the interior of the container which is at least partly filled with the cosmetic preparation,
b3) a heating device for heating, under increased pressure, the cosmetic preparation that is located in the closed interior of the container, and releasing, under reduced pressure, the heated cosmetic preparation from the interior of the container into the surroundings,
b4) a nozzle which allows atomization of the cosmetic preparation escaping from the container,
c) a storage container for the cosmetic preparation, from which the cosmetic preparation can enter the container, wherein
- the access between the storage container and the container has a component for flow control, by means of which component the flow of the cosmetic preparation from the storage container into the container can be interrupted,
- the storage container has at least ten times the volume, preferably at least twenty times and in particular at least fifty times the volume of the container, and
- and the pressure inside the storage container corresponds to the ambient pressure,
a cosmetic preparation a) containing, based on the total weight thereof,
a1) at least 88 wt.% polar solvent;
a2) at least 0.1 wt.% film-forming polymer,
is applied to the keratin-containing fibers,
wherein the proportion by weight of components a1) and a2) with respect to the total weight of the cosmetic preparation is more than 92%.

11. The method according to claim 10, **characterized in that**
- some of the cosmetic preparation located in the storage container is transferred from said storage container into the container;
- the access between the storage container and the container is subsequently interrupted by means of a component for flow control, by means of which the flow of the cosmetic preparation from the storage container into the container can be interrupted;
- the cosmetic preparation located in the container, which is sealed against the surroundings, is subsequently heated by means of a heating device such that the pressure inside the container increases to values above the ambient pressure, preferably to values between 1.1 and 8 bar, in particular to values between 1.2 and 4 bar;
- the container, which is pressurized above the ambient pressure, is subsequently opened in a manner in which the discharge of at least some, preferably at least 50 wt.%, more preferably at least 80 wt.%, and in particular at least 90 wt.%, of the cosmetic preparation located in the container is released from the container into the surroundings by the pressure that prevails in the container at the time at which the container is opened being reduced.

## Revendications

1. Produit cosmétique, comprenant
a) une préparation cosmétique contenant, par rapport à son poids total,
a1) au moins 88 % en poids d'un solvant polaire ;
a2) au moins 0,1 % en poids d'un polymère filmogène ;
le pourcentage en poids des constituants a1) et a2) par rapport au poids total de la préparation cosmétique a) étant supérieur à 92 %
b) un dispositif d'évaporation flash de la préparation cosmétique, comportant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de la préparation cosmétique,
b3) un dispositif de chauffage permettant de chauffer la préparation cosmétique présente dans l'espace intérieur fermé du récipient tout en augmentant la pression, et de détendre la préparation cosmétique chauffée hors de l'espace intérieur du récipient tout en réduisant la pression dans l'environnement,
b4) une buse permettant d'atomiser la préparation cosmétique s'échappant du récipient,
c) un récipient de stockage destiné à la préparation cosmétique, à partir duquel la préparation cosmétique peut pénétrer dans le récipient,
- l'accès entre le récipient de stockage et le récipient présentant un élément de régulation de flux, au moyen duquel le flux de la préparation cosmétique depuis le récipient de stockage jusqu'au récipient peut être interrompu,
- le récipient de stockage présentant au moins dix fois le volume, de préférence au moins vingt fois et en particulier au moins cinquante fois le volume du récipient,
- la pression à l'intérieur du récipient de stockage correspondant à la pression ambiante.

2. Produit cosmétique selon la revendication 1, **caractérisé en ce que** le pourcentage en poids du solvant polaire a1) par rapport au poids total de la préparation cosmétique est de 88 à 99 % en poids, de préférence de 90 à 98 % en poids et en particulier de 92 à 97 % en poids.

3. Produit cosmétique selon l'une des revendications précédentes, **caractérisé en ce que** le solvant polaire a1) est choisi dans le groupe constitué par l'éthanol et l'eau.

4. Produit cosmétique selon l'une des revendications précédentes, **caractérisé en ce que** le pourcentage en poids du polymère filmogène a2) par rapport au poids total de la préparation cosmétique est de 0,1 à 10 % en poids, de préférence de 0,2 à 8,0 % en poids et en particulier de 0,4 à 5,0 % en poids.

5. Produit cosmétique selon l'une des revendications précédentes, **caractérisé en ce que** le polymère filmogène a2) est choisi dans le groupe des polymères non ioniques, de préférence dans le groupe des polyvinylpyrrolidones et des copolymères vinylpyrrolidone/acétate de vinyle, en particulier des copolymères vinylpyrrolidone/acétate de vinyle.

6. Produit cosmétique selon l'une des revendications précédentes, **caractérisé en ce que** le polymère filmogène a2) est choisi dans le groupe des polyuréthanes, de préférence dans le groupe des polymères polyuréthannes anioniques, comprenant au moins un motif structural de formule (I) et au moins un motif structural de formule (II) et au moins un motif structural de formule (III) et au moins un motif structural de formule (IV) et au moins un motif structural de formule (V) : 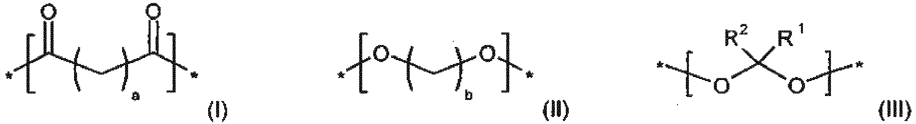 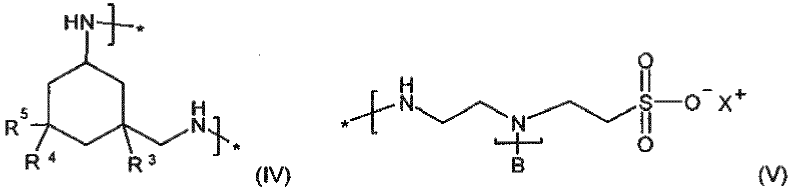 dans lesquelles
- a et b représentent, indépendamment l'un de l'autre, des nombres entiers allant de 1 à 20,
- R¹, R², R³, R⁴ et R⁵ représentent chacun, indépendamment l'un de l'autre, un atome d'hydrogène ou un groupe alkyle en C₁-C₄,
- B représente un atome d'hydrogène ou une liaison directe avec un autre motif structural,
- X⁺ représente un cation physiologiquement compatible.

7. Utilisation d'une préparation cosmétique contenant, par rapport à son poids total,
a) une préparation cosmétique contenant, par rapport à son poids total,
a1) au moins 88 % en poids d'un solvant polaire ;
a2) au moins 0,1 % en poids d'un polymère filmogène,
le pourcentage en poids des constituants a1) et a2) par rapport au poids total de la préparation cosmétique étant supérieur à 92 %, en tant que matériau de traitement dans un dispositif d'évaporation flash de la préparation cosmétique comportant
b1) un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de la préparation cosmétique,
b3) un dispositif de chauffage permettant de chauffer la préparation cosmétique présente dans l'espace intérieur fermé du récipient tout en augmentant la pression, et de détendre la préparation cosmétique chauffée hors de l'espace intérieur du récipient tout en réduisant la pression dans l'environnement,
b4) une buse permettant d'atomiser la préparation cosmétique s'échappant du récipient,
c) un récipient de stockage destiné à la préparation cosmétique, à partir duquel la préparation cosmétique peut pénétrer dans le récipient,
- l'accès entre le récipient de stockage et le récipient présentant un élément de régulation de flux, au moyen duquel le flux de la préparation cosmétique depuis le récipient de stockage jusqu'au récipient peut être interrompu,
- le récipient de stockage présentant au moins dix fois le volume, de préférence au moins vingt fois et en particulier au moins cinquante fois le volume du récipient,
- la pression à l'intérieur du récipient de stockage correspondant à la pression ambiante.

8. Utilisation d'un produit selon l'une des revendications 1 à 6 pour l'application d'une préparation cosmétique a) sur des fibres kératiniques, en particulier des cheveux humains.

9. Utilisation d'un produit selon l'une des revendications 1 à 6 pour la déformation temporaire de fibres kératiniques, en particulier de cheveux humains.

10. Procédé de déformation temporaire de fibres kératiniques, en particulier de cheveux humains, selon lequel on applique sur les fibres kératiniques, au moyen d'un dispositif d'évaporation par détente d'une préparation cosmétique comportant b1)
un récipient définissant un espace intérieur fermé dans lequel la préparation cosmétique peut être reçue,
b2) une soupape ou un élément de fermeture à action comparable permettant de fermer et d'ouvrir l'espace intérieur du récipient rempli au moins partiellement de la préparation cosmétique,
b3) un dispositif de chauffage permettant de chauffer la préparation cosmétique présente dans l'espace intérieur fermé du récipient tout en augmentant la pression, et de détendre la préparation cosmétique chauffée hors de l'espace intérieur du récipient tout en réduisant la pression dans l'environnement,
b4) une buse permettant d'atomiser la préparation cosmétique s'échappant du récipient,
c) un récipient de stockage destiné à la préparation cosmétique, à partir duquel la préparation cosmétique peut pénétrer dans le récipient,
- l'accès entre le récipient de stockage et le récipient présentant un élément de régulation de flux, au moyen duquel le flux de la préparation cosmétique depuis le récipient de stockage jusqu'au récipient peut être interrompu,
- le récipient de stockage présentant au moins dix fois le volume, de préférence au moins vingt fois et en particulier au moins cinquante fois le volume du récipient,
- la pression à l'intérieur du récipient de stockage correspondant à la pression ambiante, une préparation cosmétique a) contenant par rapport à son poids total
a1) au moins 88 % en poids d'un solvant polaire ;
a2) au moins 0,1 % en poids d'un polymère filmogène,
le pourcentage en poids des constituants a1) et a2) par rapport au poids total de la préparation cosmétique étant supérieur à 92 %.

11. Procédé selon la revendication 10, **caractérisé en ce que**
- une fraction de la préparation cosmétique présente dans le récipient de stockage est transférée de ce récipient de stockage au récipient ;
- on interrompt ensuite l'accès entre le récipient de stockage et le récipient par un élément de régulation de flux, au moyen duquel le flux de la préparation cosmétique depuis le récipient de stockage jusqu'au récipient peut être interrompu ;
- on chauffe ensuite la préparation cosmétique présente dans le récipient isolé de l'environnement au moyen d'un dispositif de chauffage, de sorte que la pression à l'intérieur du récipient dépasse la pression ambiante et atteint de préférence des valeurs comprises entre 1,1 et 8 bar, en particulier entre 1,2 et 4 bar ;
- le récipient se trouvant à une pression supérieure à la pression ambiante est ensuite ouvert de manière à détendre au moins une fraction, de préférence au moins 50 % en poids, de façon préférée au moins 80 % en poids et en particulier au moins 90 % en poids de la préparation cosmétique présente dans le récipient et à provoquer sa sortie dans l'environnement tout en réduisant la pression régnant dans le récipient au moment de l'ouverture de celui-ci.
